# EUROPEAN PATENT SPECIFICATION

(11) **EP 2 138 133 B1**
(45) Date of publication and mention of the grant of the patent: **08.05.2019**
(21) Application number: 07858256.6
(22) Date of filing: 26.11.2007
(51) Int. Cl.: A61F 2/28, A61F 2/78

(54) **MODULAR FEMORAL ENDOPROSTHESIS**
MODULARE FEMUR-ENDOPROTHESE
ENDOPROTHÈSE FÉMORALE MODULAIRE

(30) Priority: 02.02.2007 ES 200700287
(43) Date of publication of application: 30.12.2009
(73) Proprietor: Tequir, S.L., 46190 Riba Roja del Turia (ES)
(72) Inventor: GUIRAO CANO, Luis, 46190 Riba Roja del Turia (ES); COSTA SANTOS, Carlos, 46190 Riba Roja del Turia (ES); BRESO PERIS, María Magdalena, 46190 Riba Roja del Turia (ES)
(74) Representative: ABG Intellectual Property, S.L.
(86) International application number: PCT/ES2007/000679
(87) International publication number: WO 2008/092967

(56) References cited:
- ES-T3- 2 181 427
- ES-T3- 2 245 989
- ES-T3- 2 246 448
- ES-T3- 2 265 132
- JP-A- 01 085 645
- US-A- 3 947 897
- US-A- 4 158 895
- US-A1- 2005 102 038
- US-B1- 6 485 522

## Description

### Object of the Invention

The present invention relates to a novel modular femoral endoprosthesis providing a prosthetic solution to favor the comfort of these patients, improve their quality of life and the efficacy in walking by means of a femoral endoprosthesis with a condyle-shaped end which allows distal support and suppressing ischial pressure, which allows making maximum use of the features of the socket without proximal discomfort. This endoprosthesis is anchored within the medullary cavity of the patient's femur, being able to be placed in the primary intervention (when the amputation occurs) or secondary intervention (in a second intervention), according to each patient.

Its field of application is the field of surgical interventions for endoprostheses and the manufacture thereof.

### Background of the Invention

After femoral amputation, the patient has physical and psychological sequelae which must be treated. Physical sequelae will respond to the placing a prosthesis and psychological sequelae may require specialized treatment. The adaptation to a physical handicap, such as an amputation, depends on a large variety of social, psychological and environmental factors. From a psychological point of view, the response of a person to a handicap is related to his or her psychological functioning before his or her amputation.

The prosthetic lower limb components used in the first phase can be divided into:
- socket
- endoskeletal structure
- knee (amputation above the knee)
- foot

Sockets are always annoying due to the supports necessary for the good securing thereof to the patient's stump and depend on the amputation level:
- femoral level: the patient puts his or her weight on the ischium and very little on the rest of the stump. This involves a discomfort for the patient while walking, whichever the type of socket, and a lower control of the stump since the femur is immersed in a "magma" of soft tissues. Given that the suitable amputation level is the middle third, there is enough space for placing the prosthetic knee.
- knee disarticulation level: due to the direct distal support of the stump in the socket and the weight transfer in the femoral condyles, an ischial support is not necessary, whereby it does not have the discomfort in the proximal part of the stump. The mobility of the hip joint is not restricted, unlike in the femoral level, being able to prevent local pain or pain at a lumbar level. It offers greater comfort in its use due to the fact that the support and the large surface of contact between the stump and the socket make the pressures be uniformly distributed. This level would be the most suitable one for the patient given that it has a larger lever arm than the femoral level and the support is distal and not proximal, but has the drawback that while placing the knee there is an extension of the amputated limb in the seated position.

Currently, a customized stem is inserted in the femur and a system for the connection to the artificial leg is placed in the end coming out of the stump, but this system does not have a previous first-phase assembly nor is it modular, itis customized, expensive and has a long consolidation time.

Document US 2005/0102038 A1 discloses a subcutaneous, intramuscular bearing for a rigid transcutaneous implant, for anchoring intracorporally in a bone stump and having an intermediate piece between the implant and an extracorporal coupling for coupling on the implant, wherein a bushing is connected to the intermediate piece, such that between the wall of the bushing and the intermediate piece an annular space is formed, which is closed in the intracorporal direction, for receiving and setting the extracorporal coupling.

Document US6485522 discloses an adapter for an exoprosthetic element comprising a proximal stem part installed into a long bone stump; an open-mesh three-dimensional space-network structure covering, at least partially, said stem part; a coupling device provided on a distal end of said stem part for coupling with said exoprosthetic standard part; wherein said coupling device includes a cone, an adapter sheath having a conical clamping seat, and a coupling part for connecting to said exoprosthetic standard part, said cone being seated in said conical clamping seat.

Document US 4,158,895 discloses an anchor of an artificial prosthesis in the stump, specifically in upper limbs.

Document US 3,947,897 discloses an anchor of an artificial prosthesis directly to a terminal which would already be inserted in the medullary cavity of the patient's stump.

### Description of the Invention

The present invention provides a modular femoral endoprosthesis according to claim 1.

For the purpose of solving the previously described problems, the present invention proposes the two-phase placement of a modular femoral endoprosthesis, having an outer pin from which the prosthetic knee can be hung and thus the use of a socket is avoided. This endoprosthesis would also allow the sensory perception by the patient of the so-called osseoperception or bone perception, which helps the patient to know where he or she places the prosthesis while walking.

It is a type of modular endoprosthesis, with a first surgical intervention in which the femoral condyle-shaped endoprosthesis is implanted in the patient and in patients needing a second intervention, a modular pin is implanted which allows the patient to move without needing a socket, an artificial leg being connected to the pin.

The candidates for this type of endoprosthesis are:

### 1^{st} Phase (condylar endoprosthesis)

Useful in all patients amputated at femoral level with walking capacity and without bone contraindications (e.g., severe osteoporosis) or due to a short stump, either with a vascular origin (the largest group) or a trauma / tumor / infectious origin.

### 2^{nd} Phase (endoprosthesis with a modular intramedullary pin)

Useful in those patients in whom the 1st phase is implanted and who have sufficient moving and functional capacity to walk outside his or her home (K 3 level).

With the modular femoral endoprosthesis object of the present invention a better level of quality if life is achieved for the patient since it consists of combining the femoral and knee disarticulation levels, thus having the advantages of the space allowing in the femoral level the placement of the prosthetic knee and the comfort of the distal support of the stump in the femoral condyles and the lever arm in the· knee disarticulation level, as well as the preservation or improvement of the bone density quality of the femur due to the stimulation thereof by means of the prosthesis.

The walking success of the amputated patient depends especially on the good adaptation of the socket on one hand and on the rehabilitating treatment on the other. The forces acting on the stump - prosthesis interface are an essential part of the success in the adaptation and alignment of the prosthesis.

In short, the free mobility of the hip joint allows the thigh musculature to be enhanced more efficiently than in the femoral level, and the effective weight transfer in the femoral condyles allows the patient with a disarticulated knee to walk more comfortably and with a more favorable energy expenditure.

In femoral level patients, the placement of a femoral endoprosthesis with a condyle-shaped end allows distal support and decrease the ischial pressure, which allows making maximum use of the features of the socket without proximal discomfort.

This endoprosthesis is anchored within the medullary cavity of the patient's femur, being able to be placed in the primary intervention (when the amputation occurs) or secondary intervention (in a second intervention), according to each patient. When the distance of the stump of femoral amputees increases, the area to accommodate mid-lateral stabilization forces is larger, therefore stump-socket contact pressures are lower.

All patients with a femoral amputation level are susceptible to the placement of this type of endoprosthesis, whatever their age. With the comfort that the patient would obtain with the distal support, he or she could use the prosthesis for more time and with more quality.

Therefore, the patient obtains the following improvements with respect to what exists to date:
- Better control of the prosthetic knee since he or she can put his or her weight distally, in relation to the femoral level which does not support. The consequence will be having greater stability and safety while moving.
- Better control of the stump whereby the patient is more stable while moving.
- Improvement of the movement in the Trendelenburg position due to the control of the socket, with lower energy expenditure in the movement.
- It allows changes in the socket:
   - Possibility of making front and rear windows which provide greater comfort to the patient in the seated position.
   - Suppression of the pressure in the ischium, therefore the patient can walk without so much discomfort.
   - Bone stimulation caused by the prosthesis favoring the preservation of a good bone density and preventing femoral neck fracture problems derived from the absence of loads in this type of patient.

### Description of the Drawings

To complement the description which is being made and with the aim of aiding to better understand the features of the invention, a series of figures are attached to the present specification as an integral part thereof, in which the following has been depicted with an illustrative and non-limiting character:

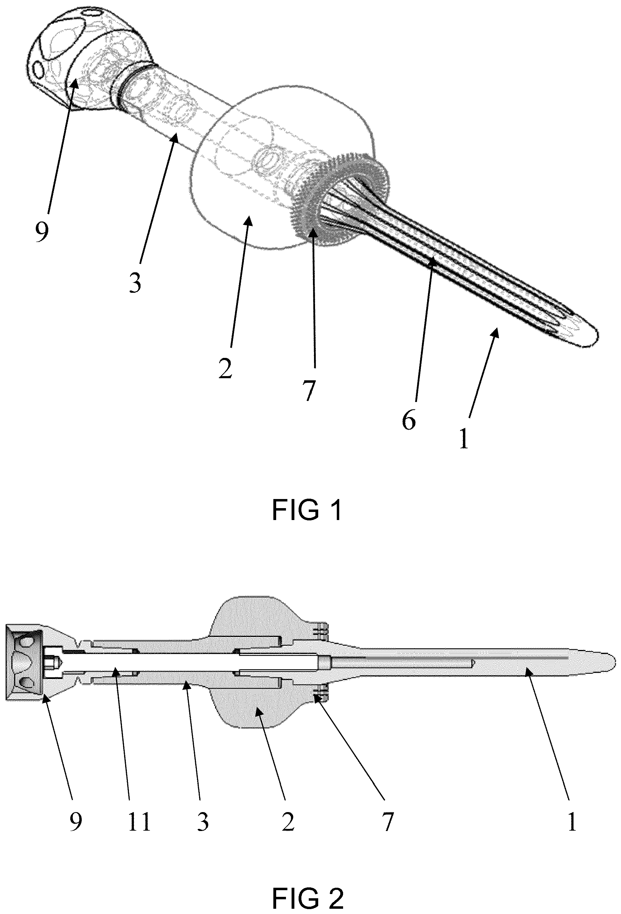

Figure 1 shows an upper side perspective wireframe view of a practical embodiment of the endoprosthesis object of the present invention assembled in the second phase.
Figure 2 shows a section view of the previous endoprosthesis.

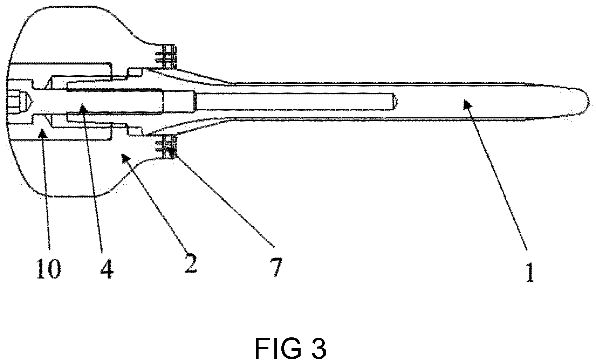

Figure 3 shows a view of the assembly assembled in the first phase.

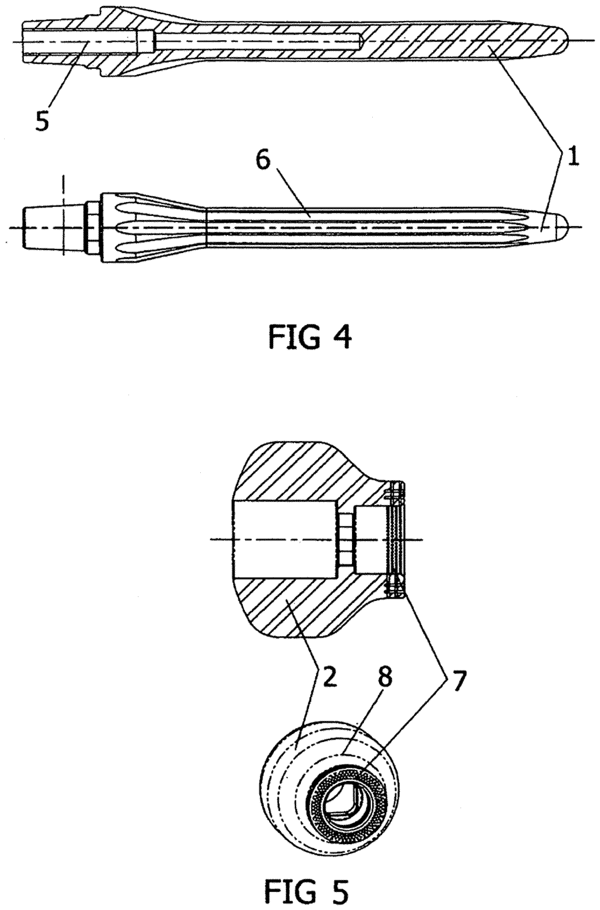

Figure 4 shows a longitudinal and sectioned view of the proximal stem.
Figure 5 shows a spacer.

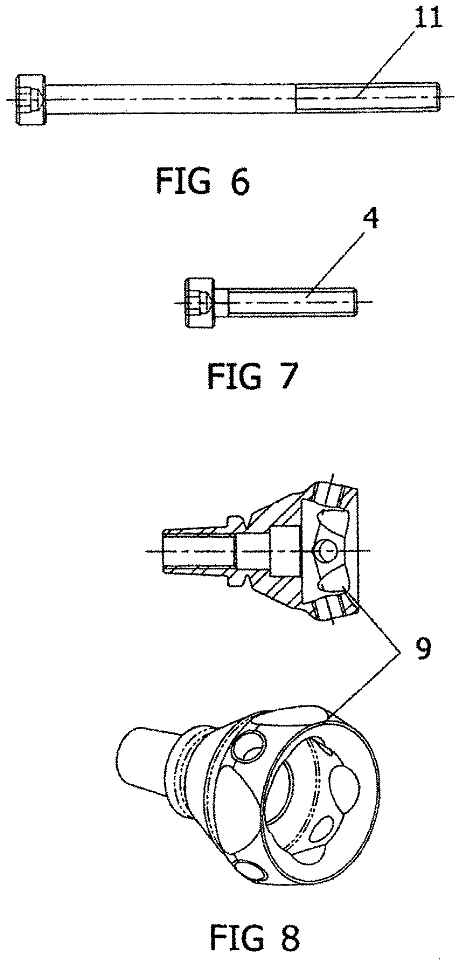

Figure 6 shows a closing screw.
Figure 7 shows a short locking screw.
Figure 8 shows a pyramidal socket.

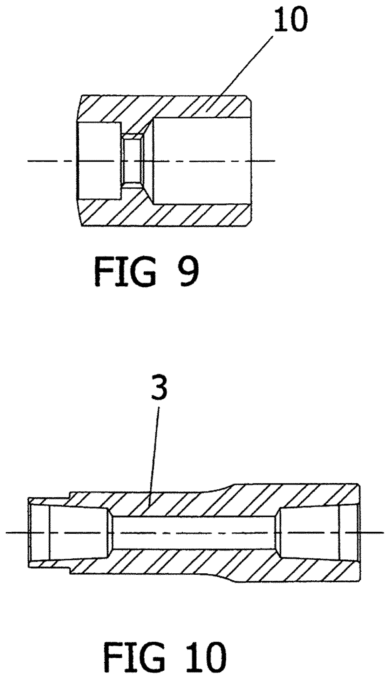

Figure 9 shows a closing cap.
Figure 10 shows an extending tube.

### Preferred Embodiment of the Invention

The attached figures first show a proximal stem (1), which can have several measurements, as well as a spacer (2) with a damping and integrating system, a locking cylinder (3) and a short closing screw (4).

The proximal stem (1) is formed with a preferably grooved body (6) and is provided with a threaded hole (5) where the closing screw (4) is inserted. In addition, the spacer is coupled to the extending cylinder (3) such that it envelops the attachment of this cylinder and the stem (1), and has a series of holes (7).
- In the first phase, the spacer (2) is made of a polymer having a modulus of elasticity similar to that of the bone.
- In the area of contact with the front of the femur it has holes (7) ensuring a good osseointegration for its secondary fixing and damping of the impact.
- The closing cap (10) made of the same material as the spacer serves to fix the spacer (2) to the stem (1) and protect the conical adjustment for the second phase.
- It is provided with an extraction system to facilitate its removal for the second phase.
- It is fixed to the stem with a short titanium alloy screw (4).

The stem (1) is made of titanium alloy for small diameters and for larger diameters it is made of PEEK which ensures certain elasticity. These stems can be coated with a biointegrating material and have different lengths and diameters according to the case.

For the second phase, a titanium alloy adapter (9) with a standard orthoprosthesis terminal is used and it has a brittleness groove (8) for controlled breakage in the event of an accident or overload.
- The alloy extender (3) is made of titanium with a size variable according to the case, and attaches the adapter to the stem (1)
- The long screw (11) preferably made of pure titanium (more plastic than the titanium alloy) ensures the fixing of the assembly with the possibility of being deformed in the event of breakage of the adapter, but without being broken.

Its application and operation is very simple, to that end, in the first phase the assembly is done outside the area and the stem (1) assembled with the spacer (2), the closing cap (10) and the short locking screw (4) is inserted. This is the basic prosthesis.

If the user wishes to, in a second phase of this system, an incision is made in the stump, the short locking screw (4) and the closing cap (10) are removed. The modularity is achieved with the elongated tube (3) which is assembled with the adapter with the terminal standard (9) for an artificial prosthesis and the assembly is locked with the long screw (11).

Having sufficiently described the nature of the present invention as well as a practical application thereof, it must only be added that its shape and materials as well as the implementation thereof are susceptible to modifications, provided that they do not substantially affect the features that are claimed below:

## Claims

1. A modular femoral endoprosthesis, used in femoral amputations, comprising:
a proximal stem (1) having a grooved body (6) and a threaded hole (5), and
a spacer (2) with a damping and integrating system, wherein the spacer (2) has a series of holes (7) in the area intended to be in contact with the front of the femur, favoring osseointegration and damping;
wherein the endoprosthesis has a condyle-shaped end; and
wherein the endoprosthesis further comprises:
A)
a short closing screw (4) adapted to be screwed into the threaded hole (5) of the proximal stem (1) so as to secure the spacer (2) and a closing cap (10) to the proximal stem (1), when the endoprosthesis is intended to be used for a first phase, or
B)
a long screw (11) adapted to be screwed into the threaded hole (5) of the proximal stem (1) so as to secure a locking modular extending cylinder (3), and an adapter with a prosthesis connector terminal (9) to the proximal stem (1), when the endoprosthesis is intended to be used for a second phase.

2. The modular femoral endoprosthesis according to claim 1, wherein the spacer (2) is coupled to the extending cylinder (3) such that it envelops the attachment of this extending cylinder and the stem (1) when the endoprosthesis is intended to be used for a second phase.

3. The modular femoral endoprosthesis according to claim 1, wherein the closing cap (10) is made of the same material as the spacer (2) and fixes this spacer (2) to the stem (1), protecting the conical adjustment for the second phase.

4. The modular femoral endoprosthesis according to claim 1, wherein the titanium alloy adapter (9) with an orthoprosthesis terminal has a brittleness groove (8) for controlled breakage.

## Patentansprüche

1. Modulare Femurendoprothese zur Verwendung bei Femuramputationen, mit:
einem proximalen Schaft (1), der einen gefurchten Korpus (6) und ein Gewindeloch (5) aufweist, und
einem Abstandsstück (2) mit einem Dämpfungs- und Integrationssystem, wobei das Abstandsstück (2) eine Reihe von Löchern (7) in dem Bereich, der mit der Vorderseite des Femurs in Kontakt gebracht werden soll, zur Begünstigung der Osseointegration und Dämpfung aufweist;
wobei die Endoprothese ein Kondylus-förmiges Ende aufweist; und
wobei die Endoprothese ferner umfasst:
A)
eine kurze Verschlussschraube (4), die dazu ausgebildet ist, in das Gewindeloch (5) des proximalen Schafts (1) geschraubt zu werden, um das Abstandsstück (2) und eine Verschlusskappe (10) an dem proximalen Schaft (1) zu sichern,
wenn die Endoprothese für eine erste Phase verwendet werden soll, oder
B)
eine lange Schraube (11), die dazu ausgebildet ist, in das Gewindeloch (5) des proximalen Schafts (1) geschraubt zu werden, um einen sich als Modul erstreckenden Verriegelungszylinder (3), und einen Adapter mit einem Prothesen-Verbindungsende (9) an dem proximalen Schaft (1) zu sichern, wenn die Endoprothese für eine zweite Phase verwendet werden soll.

2. Modulare Femurendoprothese nach Anspruch 1, wobei das Abstandsstück (2) mit dem sich erstreckenden Zylinder (3) verbunden ist, so dass es die Befestigung dieses sich erstreckenden Zylinders und des Schafts (1) umgibt, wenn die Endoprothese für eine zweite Phase verwendet werden soll.

3. Modulare Femurendoprothese nach Anspruch 1, wobei die Verschlusskappe (10) aus demselben Material wie das Abstandsstück (2) gefertigt ist und dieses Abstandsstück (2) an dem Schaft (1) befestigt, um die konische Anpassung für die zweite Phase zu schützen.

4. Modulare Femurendoprothese nach Anspruch 1, wobei der Adapter (9) aus Titanium-Legierung mit einem Ortoprothesenende eine Sprödigkeitsnut (8) zum kontrollierten Bruch aufweist.

## Revendications

1. Une endoprothèse fémorale modulaire, utilisée pour les amputations fémorales, comprenant :
une tige proximale (1) ayant un corps rainuré (6) et un trou fileté (5), et
une pièce d'espacement (2) avec un système d'amortissement et d'intégration, la pièce d'espacement (2) ayant une série de trous (7) dans la zone destinée à être en contact avec la partie frontale du fémur, favorisant une ostéointégration et un amortissement ;
l'endoprothèse ayant une extrémité en forme de condyle ; et
l'endoprothèse comprenant en outre :
A)
une vis de fermeture courte (4) adaptée pour être vissée dans le trou fileté (5) de la tige proximale (1) afin de fixer la pièce d'espacement (2) et un capuchon de fermeture (10) à la tige proximale (1), lorsque l'endoprothèse est destinée à être utilisée pour une première phase, ou
B)
une vis longue (11) adaptée pour être vissée dans le trou fileté (5) de la tige proximale (1) afin de fixer à la tige proximale (1) un cylindre d'extension (3) verrouillable et modulaire, et un adaptateur avec une partie terminale (9) formant connecteur de prothèse, lorsque l'endoprothèse est destinée à être utilisée pour une deuxième phase.

2. L'endoprothèse fémorale modulaire selon la revendication 1, dans laquelle la pièce d'espacement (2) est reliée au cylindre d'extension (3) de telle sorte qu'elle enveloppe la partie de fixation de ce cylindre d'extension et de la tige (1) lorsque l'endoprothèse est destinée à être utilisée pour une deuxième phase.

3. L'endoprothèse fémorale modulaire selon la revendication 1, dans laquelle le capuchon de fermeture (10) est réalisé dans le même matériau que la pièce d'espacement (2) et fixe cette pièce d'espacement (2) à la tige (1), protégeant ainsi le réglage conique pour la deuxième phase.

4. L'endoprothèse fémorale modulaire selon la revendication 1, dans laquelle l'adaptateur (9) en alliage de titane avec une partie terminale d'orthoprothèse présente une rainure de fragilité (8) pour une rupture contrôlée.
